# EUROPEAN PATENT APPLICATION

(11) **EP 4 789 712 A1**
(43) Date of publication of application: **12.08.2026**
(21) Application number: 26153979.5
(22) Date of filing: 26.01.2026
(51) Int. Cl.: A61N 1/05, A61N 1/375, A61N 1/372

(54) **BIOSTIMULATOR HAVING ANTI-DISLODGEMENT FEATURES**

(30) Priority: 28.01.2025 US 202563750728 P; 23.01.2026 US 202619458577
(71) Applicant: Pacesetter, Inc., Sylmar, CA 91342 (US)
(72) Inventor: CHANTASIRIVISAL, Steve, Sylmar, CA 91342 (US); VICTORINE, Keith, Sylmar, CA 91342 (US); ALLEMAN, Wesley, Sylmar, CA 91342 (US); TEAGUE, Bryan, Sylmar, CA 91342 (US); LEDBETTER, Cody, Sylmar, CA 91342 (US); WELCH, Mark, Sylmar, CA 91342 (US); FISHLER, Matthew G, Sylmar, CA 91342 (US); NIX, Kyle J, Sylmar, CA 91342 (US)
(74) Representative: Barker Brettell Sweden AB

(57) **Abstract**

A biostimulator (100) includes a housing (302) having a longitudinal axis (304) and containing an electronics compartment (306). A fixation element mount (311) is mounted on the housing (302) and a fixation element (106) is mounted on the fixation element mount (311). The fixation element (106) extends helically about the longitudinal axis (304) and has an outer dimension (902). The outer dimension (902) increases in a distal direction (408) over a proximal section (904) of the fixation element (106), and the outer dimension (902) decreases in the distal direction (408) over a distal section (906) of the fixation element (106).

## Description

### TECHNICAL FIELD

The present disclosure relates to biostimulators. More specifically, the present disclosure relates to leadless biostimulators useful for deep septal pacing.

### BACKGROUND

Cardiac pacing by an artificial pacemaker provides an electrical stimulation of the heart when its own natural pacemaker and/or conduction system fails to provide synchronized atrial and ventricular contractions at rates and intervals sufficient for a patient's health. Such antibradycardial pacing provides relief from symptoms and even life support for hundreds of thousands of patients. Cardiac pacing may also provide electrical overdrive stimulation to suppress or convert tachyarrhythmias, again supplying relief from symptoms and preventing or terminating arrhythmias that could lead to sudden cardiac death.

Leadless cardiac pacemakers incorporate electronic circuitry at the pacing site and eliminate leads, thereby avoiding shortcomings associated with conventional cardiac pacing systems. Leadless cardiac pacemakers can be anchored at the pacing site, e.g., in a right ventricle and, for dual-chamber pacing, in a right atrium, by an anchor.

Cardiac pacing of the His-bundle is clinically effective and advantageous by providing a narrow QRS affecting synchronous contraction of the ventricles. His-bundle pacing in or near a membranous septum of a heart, however, has some drawbacks. The procedure is often long in duration and requires significant fluoroscopic exposure. Furthermore, successful His-bundle pacing cannot always be achieved. Pacing thresholds are often high, sensing is challenging, and success rates can be low.

Deep septal pacing is an alternative to His-bundle pacing. Deep septal pacing involves pacing past the His-bundle toward the right ventricle apex. More particularly, deep septal pacing targets the left bundle branch below the His site. Deep septal pacing has been achieved using a lead in which the electrode penetrates several millimeters into the septum. Pacing thresholds associated with deep septal pacing are potentially lower than with His-bundle pacing, and clinical efficacy of the approach has been demonstrated.

### SUMMARY

Deep septal pacing, e.g., left bundle branch area pacing (LBBAP) can require a pacing electrode to penetrate through a majority of a ventricular septal wall to extend into the left bundle branch or into a left bundle fascicular that resides on a left side of a septum. In the case of a leadless cardiac pacemaker, a body of the pacemaker can be affixed to the septum and the pacing electrode can penetrate to the target tissue. More particularly, the pacing electrode may be required to penetrate 10-12 mm into the ventricular septal wall. Affixation of the leadless cardiac pacemaker to the septal wall and a depth of the pacing electrode should be accurately performed for effective treatment.

Existing leadless cardiac pacemakers are not well suited to ensuring affixation of the leadless cardiac pacemaker to the septal wall. A size and weight of leadless cardiac pacemakers required to house the requisite battery and circuitry can create a dislodgement risk using fixation elements in pliable tissue. Accordingly, fixation features to aid in reducing dislodgement of pacemaker systems, e.g., pacing leads and/or leadless cardiac pacemakers, could be useful.

A biostimulator is described. The biostimulator includes a housing having a longitudinal axis and containing an electronics compartment. A pacing element is coupled to the housing. The pacing element includes a conductor extending along the longitudinal axis to a pacing tip. The biostimulator includes a sheath surrounding the conductor. The sheath includes one or more protrusions extending radially outward from an outer sheath surface.

A biostimulator is described. The biostimulator includes a housing having a longitudinal axis and containing an electronics compartment. A fixation element mount is mounted on the housing. The biostimulator includes a fixation element mounted on the fixation element mount. The fixation element extends helically about the longitudinal axis and has an outer dimension. The outer dimension increases in a distal direction over a proximal section of the fixation element. The outer dimension decreases in the distal direction over a distal section of the fixation element.

A biostimulator is described. The biostimulator includes a housing having a longitudinal axis and containing an electronics compartment. A fixation element mount is mounted on the housing. The fixation element mount includes a compliant ring extending circumferentially about the longitudinal axis. The biostimulator includes a fixation element mounted on the fixation element mount.

A biostimulator is described. The biostimulator includes a housing having a longitudinal axis and containing an electronics compartment. A fixation element mount is mounted on the housing. The fixation element mount includes several flexible barbs extending in a distal direction. The biostimulator includes a fixation element mounted on the fixation element mount.

A biostimulator is described. The biostimulator includes a housing having a longitudinal axis and containing an electronics compartment. A pacing element is coupled to the housing. The pacing element includes a conductor extending along the longitudinal axis to a pacing tip. The biostimulator includes a sheath surrounding the conductor. The biostimulator includes a fixation element mount mounted on the housing. A fixation element is mounted on the fixation element mount. One or more of the sheath or the fixation element have a rough surface.

A biostimulator system is described. The biostimulator includes a biostimulator transport system and any of the above-described biostimulators mounted on the biostimulator transport system.

The above summary does not include an exhaustive list of all aspects of the present invention. It is contemplated that the invention includes all systems and methods that can be practiced from all suitable combinations of the various aspects summarized above, as well as those disclosed in the Detailed Description below and particularly pointed out in the claims filed with the application. Such combinations have particular advantages not specifically recited in the above summary.

### BRIEF DESCRIPTION OF THE DRAWINGS

The novel features of the invention are set forth with particularity in the claims that follow. A better understanding of the features and advantages of the present invention will be obtained by reference to the following detailed description that sets forth illustrative embodiments, in which the principles of the invention are utilized, and the accompanying drawings.
FIG. 1 is a diagrammatic cross section of a patient heart illustrating an example implantation of a biostimulator in a target anatomy, in accordance with an embodiment.
FIG. 2 is a perspective view of a biostimulator system, in accordance with an embodiment.
FIG. 3 is a side view of a biostimulator having a pacing element, in accordance with an embodiment.
FIG. 4 is a side view of a distal portion of a biostimulator having a pacing element, in accordance with an embodiment.
FIG. 5 is a side view of a distal portion of a biostimulator having a ramped protrusion, in accordance with an embodiment.
FIG. 6 is a perspective view of a distal portion of a biostimulator having a ramped protrusion, in accordance with an embodiment.
FIG. 7 is a side view of a distal portion of a biostimulator having a bulbous ring, in accordance with an embodiment.
FIG. 8 is a side view of a distal portion of a biostimulator having a bulbous ring, in accordance with an embodiment.
FIG. 9 is a side view of a distal portion of a biostimulator having a bulging fixation element, in accordance with an embodiment.
FIG. 10 is a side view of a distal portion of a biostimulator having a bulging fixation element, in accordance with an embodiment.
FIG. 11 is a side view of a distal portion of a biostimulator having a compliant ring, in accordance with an embodiment.
FIG. 12 is a perspective view of a distal portion of a biostimulator having a compliant ring, in accordance with an embodiment.
FIG. 13 is a side view of a distal portion of a biostimulator having a flexible barb, in accordance with an embodiment.
FIG. 14 is a side view of a distal portion of a biostimulator having a flexible barb, in accordance with an embodiment.
FIG. 15 is an end view of a distal portion of a biostimulator having a flexible barb, in accordance with an embodiment.
FIG. 16 is a side view of a distal portion of a biostimulator having a rough surface, in accordance with an embodiment.

### DETAILED DESCRIPTION

Embodiments describe a biostimulator, such as a leadless pacemaker, having an anti-dislodgment feature. As described below, the biostimulator can be used to perform deep septal pacing of a heart. The biostimulator may, however, be used in other applications, such as deep brain stimulation. Thus, reference to the biostimulator as being a cardiac pacemaker for deep septal pacing is not limiting. Furthermore, although the anti-dislodgement features are described with respect to incorporation in a biostimulator, such features may similarly benefit pacing leads, such as cardiac leads implanted deep in a septum of a heart for physiological pacing. Accordingly, the usefulness of the dislodgement features are not limited to a particular pacing device or application.

In various embodiments, description is made with reference to the figures. However, certain embodiments may be practiced without one or more of these specific details, or in combination with other known methods and configurations. In the following description, numerous specific details are set forth, such as specific configurations, dimensions, and processes, in order to provide a thorough understanding of the embodiments. In other instances, well-known processes and manufacturing techniques have not been described in particular detail in order to not unnecessarily obscure the description. Reference throughout this specification to "one embodiment," "an embodiment," or the like, means that a particular feature, structure, configuration, or characteristic described is included in at least one embodiment. Thus, the appearance of the phrase "one embodiment," "an embodiment," or the like, in various places throughout this specification are not necessarily referring to the same embodiment. Furthermore, the particular features, structures, configurations, or characteristics may be combined in any suitable manner in one or more embodiments.

The use of relative terms throughout the description may denote a relative position or direction. For example, "distal" may indicate a first direction along a longitudinal axis of a biostimulator. Similarly, "proximal" may indicate a second direction opposite to the first direction. Similarly, clockwise can refer to a first rotational direction and counterclockwise can refer to a second rotational direction opposite to the first rotational direction. Such terms are provided to establish relative frames of reference, however, and are not intended to limit the use or orientation of a biostimulator to a specific configuration described in the various embodiments below.

In an aspect, a biostimulator, e.g., a leadless pacemaker, includes anti-dislodgement features. The anti-dislodgement features can be located on a distal region of the biostimulator, or a cardiac lead. As described below, the anti-dislodgement features can center around geometric designs, coatings, surface modifications, and textures that enhance fixation of the biostimulator to a target tissue. For example, the anti-dislodgment features can be incorporated on an extended electrode, fixation helix, or helix mount to resist rotational and/or translational movement that causes dislodgment of the biostimulator. Accordingly, the anti-dislodgment features can aid in reducing dislodgement of pacemaker systems, e.g., pacing leads and/or leadless cardiac pacemakers, from target tissue.

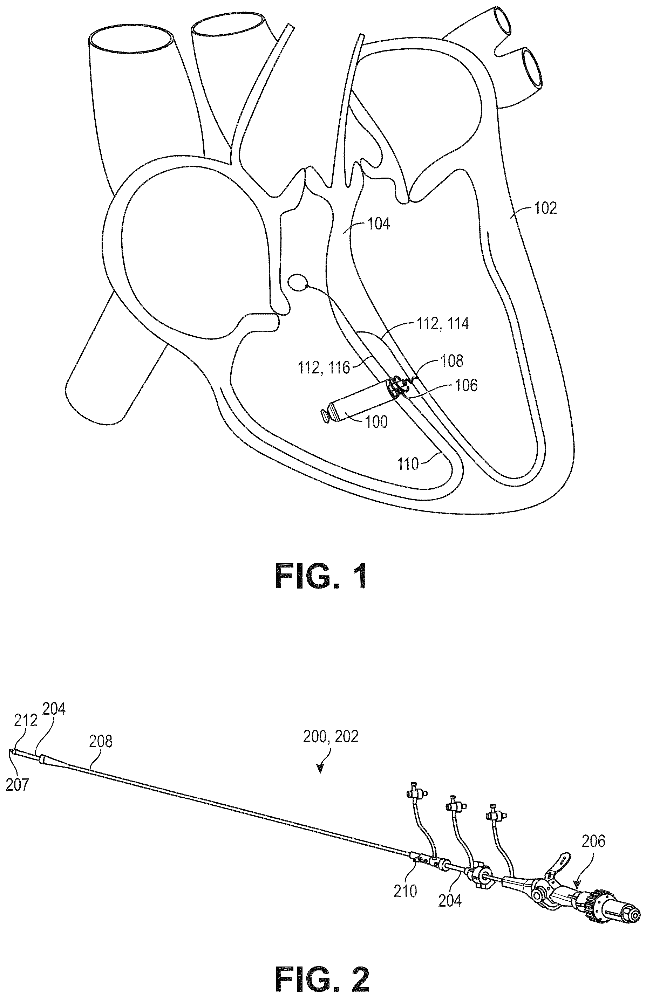

Referring to FIG. 1, a diagrammatic cross section of a patient heart illustrating an example implantation of a biostimulator in a target anatomy is shown in accordance with an embodiment. A leadless biostimulator system, e.g., a cardiac pacing system, includes one or more biostimulators 100. The biostimulator(s) 100 can be implanted in a patient heart 102, and can be leadless (and thus, may be leadless cardiac pacemakers). Each biostimulator 100 can be placed in a cardiac chamber, such as a right atrium and/or right ventricle of the heart 102, or attached to an inside or outside of the cardiac chamber. For example, the biostimulator 100 can be attached to a septum 104 of the heart 102. More particularly, the biostimulator 100 can be delivered to the septum 104, and one or more elements, such as a fixation element 106 and/or a pacing element 108 can pierce a septal wall 110 of the septum 104 to engage and anchor the biostimulator 100 to the tissue. In a particular embodiment, the biostimulator 100 can use two or more electrodes located on or within a housing of the biostimulator 100 for pacing the cardiac chamber upon receiving a triggering signal from at least one other device within the body. In an embodiment, one or more of the fixation element 106 or the pacing element 108 is an active electrode.

Leadless pacemakers or other leadless biostimulators 100 can be delivered to or retrieved from a patient using delivery or retrieval systems. The leadless biostimulator system can include delivery or retrieval systems, which may be catheter-based systems used to carry a leadless biostimulator 100 intravenously to or from a patient anatomy. The delivery or retrieval systems may be referred to collectively as transport systems. In some implementations of transport systems, a leadless pacemaker is attached or connected to a distal end of a catheter and advanced intravenously into or out of the heart 102. The transport system can include features to engage the leadless pacemaker to allow fixation of the leadless pacemaker to tissue. For example, in implementations where the leadless pacemaker includes an active engaging mechanism, such as a fixation element 106, the transport system can include a docking cap or key at a distal end of the catheter, and the docking cap or key may be configured to engage the leadless pacemaker and apply torque to screw the active engaging mechanism into or out of the tissue. In other implementations, the transport system includes clips designed to match the shape of a feature on the leadless pacemaker and apply torque to screw the active engaging mechanism into or out of the tissue.

When the biostimulator 100 is delivered to and screwed into the septum 104 of the heart 102, the pacing element 108 and/or the fixation element 106 may be positioned for deep septal pacing at respective bundle branches 112 in the septum 104. For example, an active electrode of the pacing element 108 can be positioned at the left bundle branch 114 in the septum 104. Similarly, the fixation element 106 can be positioned at or proximal to the right bundle branch 116 in the septum 104. Optionally, one of the elements may be at a bundle branch and the other element may not be at a bundle branch.

Referring to FIG. 2, a perspective view of a biostimulator system is shown in accordance with an embodiment. A biostimulator system 200 includes the biostimulator 100 (not shown) mounted on a biostimulator transport system 202. As described above, the biostimulator 100 can be delivered to and retrieved from a patient anatomy using the biostimulator transport system 202. In some implementations, the biostimulator transport system 202 is a delivery system for delivering the leadless pacemaker to the target tissue. In some implementations, the biostimulator transport system 202 is a retrieval system for retrieving the leadless pacemaker from the target tissue. The biostimulator transport system 202 can include a release mechanism to retain any of the biostimulators described below in an unreleased state and to transition into a released state to release the biostimulators into the target anatomy.

The biostimulator transport system 202 can include an elongated catheter 204 extending distally from a handle 206 to a distal catheter end 207. The elongated catheter 204 can be a deflectable catheter, and an operator can use the handle 206 to steer the distal catheter end 207 in the patient. In an embodiment, the biostimulator transport system 202 includes a guide catheter 208 mounted on the elongated catheter 204. The guide catheter 208 can be slidably disposed on the elongated catheter 204 such that a distal portion of the guide catheter 208 can slide distally over the distal catheter end 207 of the elongated catheter 204 and/or the biostimulator 100, which may be mounted on the distal catheter end (not shown). Similarly, the biostimulator transport system 202 can include an introducer hub assembly 210 mounted on the guide catheter 208. The introducer hub assembly 210 can be slidably disposed on the guide catheter 208 such that a distal portion of the introducer hub assembly 210 can slide distally over the distal catheter end 207 of the elongated catheter 204 and/or the distal portion of the guide catheter 208. More particularly, the introducer hub assembly 210 can be inserted into an access sheath to gain access to the patient vasculature, and after access is established, the distal portion of the guide catheter 208 and/or the distal catheter end 207 of the elongated catheter 204 can be advanced through the access sheath into the patient.

The distal catheter end 207 of the elongated catheter 204 may be selectively connectable to the biostimulator 100. More particularly, the biostimulator 100 can be mounted on the distal catheter end 207 of the elongated catheter 204. The biostimulator 100 can be protected by a protective sheath of the distal portion of the guide catheter 208 during delivery and/or retrieval of the biostimulator 100 from the patient. Accordingly, the biostimulator 100 can be advanced into the patient along with the distal catheter end 207.

The leadless pacemaker system can be used to implant one or more biostimulators 100 within an atrium and/or a ventricle of a heart 102 of the patient. Implantation of each biostimulator 100 may be achieved, in part, by endocardial insertion of the biostimulators 100. For example, the elongated catheter 204 of the leadless pacemaker system can include a torque shaft coupled to a docking cap 212. The docking cap 212 can have a docking cavity to receive an attachment feature of the biostimulator 100. The torque shaft can be torqueable and rotation of the torque shaft can rotate the docking cap 212, which can impart rotation to the attachment feature. Accordingly, torque can be transmitted through the torque shaft to rotate the biostimulator 100 in a first direction, e.g., clockwise. Rotating the biostimulator 100 when a fixation element 106 is in contact with the heart tissue can cause the fixation element to screw into the heart tissue and affix the biostimulator 100 to the heart tissue. Similarly, removal and retrieval of the biostimulators 100 may be accomplished endocardially. For example, the torque shaft of the elongated catheter 204 can be rotated in a second direction, e.g., counterclockwise, to transmit torque through the docking cap 212 to the attachment feature to disengage the biostimulator 100 from the heart tissue.

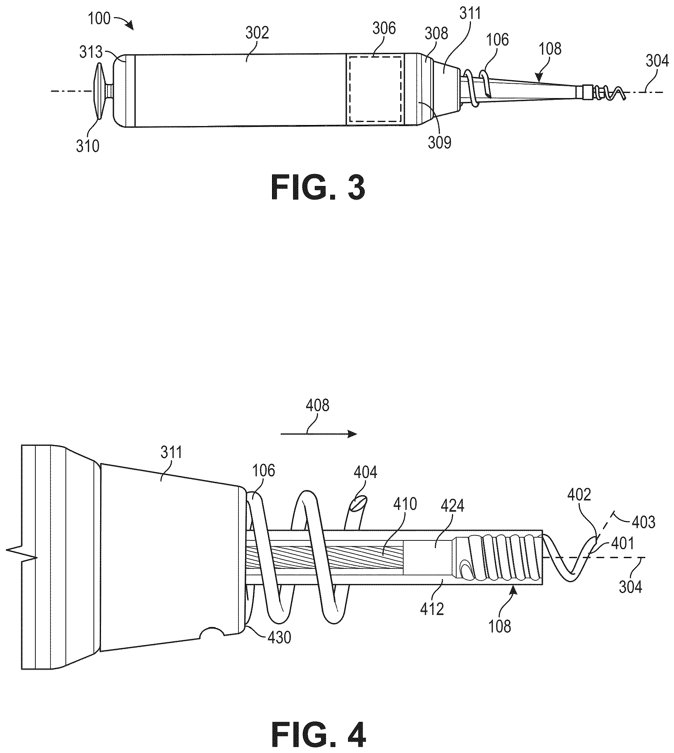

Referring to FIG. 3, a side view of a biostimulator having a pacing element is shown in accordance with an embodiment. The biostimulator 100 can be a leadless cardiac pacemaker that can perform cardiac pacing and that has many of the advantages of conventional cardiac pacemakers while extending performance, functionality, and operating characteristics. The biostimulator 100 can have two or more electrodes, e.g., a portion of a pacing element 108 that acts as an active electrode and/or a portion of the fixation element 106 or a housing 302 that acts as an active electrode. The electrodes can deliver pacing pulses to bundle branches 112 within the septum 104 of the heart 102 to perform deep septal pacing, and optionally, can sense electrical activity from the muscle. The electrodes may also communicate bidirectionally with at least one other device within or outside the body.

In an embodiment, the biostimulator 100 includes the housing 302 having a longitudinal axis 304. The housing 302 can contain a primary battery to provide power for pacing, sensing, and communication, which may include, for example, bidirectional communication. The housing 302 can optionally contain an electronics compartment 306 (shown by hidden lines) to hold circuitry adapted for different functionality. For example, the electronics compartment 306 can contain circuits for sensing cardiac activity from the electrodes, circuits for receiving information from at least one other device via the electrodes, circuits for generating pacing pulses for delivery to tissue via the electrodes, or other circuitry. The electronics compartment 306 may contain circuits for transmitting information to at least one other device via the electrodes and can optionally contain circuits for monitoring device health. The circuit of the biostimulator 100 can control these operations in a predetermined manner. The biostimulator 100 can perform leadless pacing without a pulse generator located in the pectoral region or abdomen and/or without an electrode-lead separate from the pulse generator. The biostimulator 100 may also lack a communication coil or antenna, and may not have the substantial battery power required for transmitted communication through a communication coil or antenna.

Leadless pacemakers or other leadless biostimulators 100 can be fixed to an intracardial implant site, e.g., at the septal wall 110, by one or more actively engaging mechanisms or fixation mechanisms, such as a screw or helical member that screws into the myocardium. In an embodiment, the biostimulator 100 includes the fixation element 106 coupled to the housing 302. The fixation element 106 can include a helical fixation element and/or several tines. More particularly, the biostimulator 100 can include a header assembly having a flange 308 of the housing 302. The flange 308 can be coupled to a distal housing end 309 of the housing 302. The fixation element 106 can be coupled to and extend distal to the flange 308. For example, a fixation element mount 311 can be mounted on the housing, e.g., on the flange 308, and the fixation element 106 can be mounted on the fixation element mount 311. The fixation element 106 can extend about the longitudinal axis 304. For example, in the case of a helical fixation element, the fixation element 106 can spiral about the longitudinal axis 304 to a distal fixation tip. In the case of a fixation element 106 having several tines, the tines can be arranged about the longitudinal axis and extend to respective distal tips. Accordingly, when the biostimulator 100 is delivered to the target tissue, the distal tip(s) of the fixation element 106 can pierce the tissue and the housing 302 can be rotated or pushed to affix the fixation element 106 to the target tissue.

In an embodiment, the biostimulator 100 includes an attachment feature 310. The attachment feature 310 can be mounted on a proximal housing end 313 of the housing 302. More particularly, the attachment feature 310 can be mounted on an opposite end of the housing 302 from the fixation element 106 and the pacing element 108, which as described above, can be coupled to the distal housing end 309 of the housing 302. The attachment feature 310 can facilitate precise delivery or retrieval of the biostimulator 100. For example, the attachment feature 310 can be formed from a rigid material to allow a transport system to engage the attachment feature 310 and transmit torque and/or axial loads to the attachment feature to plunge the fixation element 106 or the pacing element 108 into the target tissue.

Referring to FIG. 4, an end view of a biostimulator having a pacing element is shown in accordance with an embodiment. The biostimulator 100 can include the pacing element 108 coupled to the housing 302. The pacing element 108 can be coaxially arranged with the fixation element 106 about the longitudinal axis 304. More particularly, the pacing element 108 can extend along, e.g., axially along or helically about, the longitudinal axis 304 at a location that is radially inward from the fixation element 106.

The pacing element 108 can extend distally to a tip electrode 401. The tip electrode 401 can extend along a spiral axis 403. More particularly, the spiral axis 403 can revolve about the longitudinal axis 304, and the tip electrode 401 can extend along the spiral axis 403 to a pacing tip 402. The pacing tip 402 at a distal end of the tip electrode 401 may be distal to the distal fixation tip 404. The pacing element 108 can include a helical element to screw into a target tissue or a conical element to pierce into the target tissue. Accordingly, when the fixation element 106 screws into or otherwise engages the target tissue, the pacing element 108 can also engage the tissue, and the housing 302 can be advanced and/or rotated to cause the distal pacing tip 402 of the pacing element 108 to pierce the tissue and anchor the biostimulator 100.

One or more of the fixation element 106 or the pacing element 108 can be an active electrode, and can electrically communicate with the circuitry contained in the electronics compartment 306. Accordingly, the anchored element(s) can electrically communicate with the tissue and can transmit electrical pulses between the tissue and the circuitry of the biostimulator 100.

The biostimulator 100 can include a fixation element mount assembly that includes the fixation element 106 mounted on the fixation element mount 311. The fixation element mount 311 can be non-conductive. For example, the fixation element mount 311 can be formed from polyether ether ketone (PEEK). The fixation element 106 can include MP35N wire having a wire diameter of 0.4-0.6 mm, e.g., 0.5 mm. The wire may be formed from a metal, such as stainless steel or a nickel cobalt alloy. The wire can be wrapped into several turns to form a helical fixation element. For example, the fixation element 106 can extend helically about the longitudinal axis 304. The turns can be screwed into the septum 104 to anchor the biostimulator 100 within the heart 102.

The fixation element mount 311 can have a distal mount end 430. The distal mount end 430 may, during an operation, be pressed into the septal wall. More particularly, engagement of the distal mount end 430 with the septal wall can be indicative of a well-seated biostimulator 100, which is optimally placed for effective pacing. When the distal mount end 430 is pressed against the septal wall, the tip electrode 401 can engage the target tissue to deliver pacing pulses.

In an embodiment, the pacing element 108, which is coupled to the housing 302, extends in a distal direction 408 to the distal pacing tip 402. More particularly, the pacing element 108 can include a core assembly that extends between the electronics compartment 306 and the distal pacing tip 402. The core assembly can include one or more structural members to carry electrical pulses, such as a conductor 410. The conductor 410 may be flexible, i.e., a flexible conductor. The conductor 410 can extend in the distal direction 408, e.g., along the longitudinal axis 304, through the fixation element mount 311 and the fixation element 106 to the pacing tip 402 distal to the distal fixation tip 404.

In an embodiment, the pacing element 108 of the biostimulator 100 includes a sheath 412, e.g., an insulation sleeve surrounding, e.g., encapsulating, the conductor 410. More particularly, the sheath 412 can cover and/or surround the conductor 410. The sheath 412 can insulate a length of the pacing element 108. For example, the electrically conductive component(s) of the conductor 410 can be encapsulated by the sheath 412 over its length, including over a portion of the conductor 410 distal to the fixation element 106.

The pacing element 108 can extend distal to the fixation element mount 311 by a length sufficient for the tip electrode 401 of the pacing element 108 to access the target region, e.g., the left bundle branch 114. The left bundle branch 114 may be at a depth of 10-25 mm from the septal wall 110, typically. Accordingly, the pacing element 108 can have a length of 7-20 mm, e.g., 12 mm or 16 mm, from a distal end of the fixation element mount 311 to the distal pacing tip 402. Accordingly, the pacing element 108 can provide a means of pacing a target tissue that is distal to tissue being engaged by the fixation element 106. For example, the pacing element 108 can pace the left bundle branch 114 when the fixation element 106 is engaged near a surface of the septal wall 110 and/or near the right bundle branch 116.

The helical tip electrode 401 may be mounted on a tip support 424. The tip support 424 can include a base, which may be bonded to or crimped on to the conductor 410. A boss can extend distal to the base. The boss may be inserted into an inner channel of the distal pacing element, e.g., the helical tip electrode 401. The helical tip electrode 401 may be secured to the tip support 424, e.g., by welding the helical electrode to the base or the boss. Accordingly, the helical tip electrode 401 can be secured to the conductor 410 to receive and relay pacing impulses to the target tissue at the distal pacing tip 402.

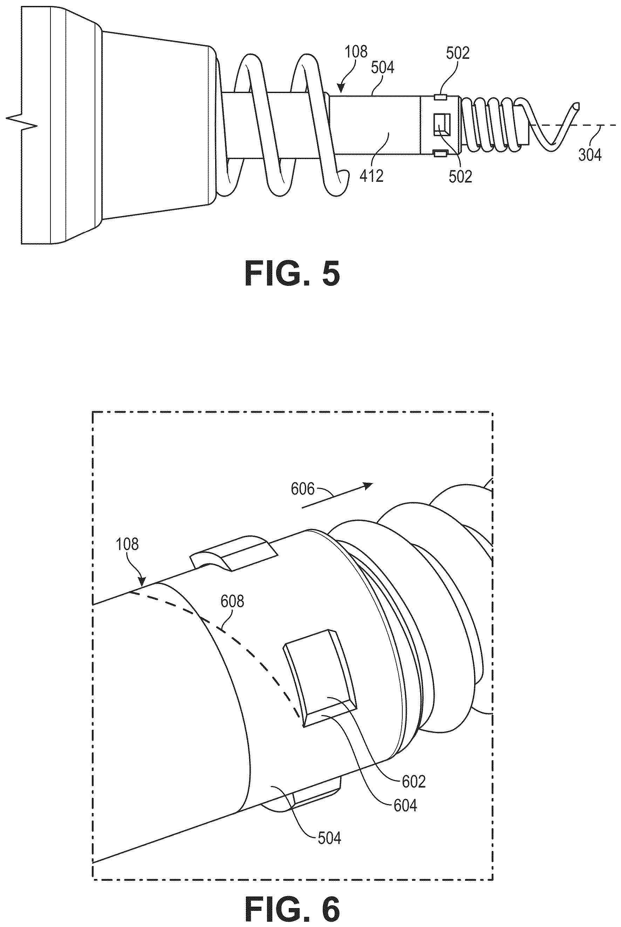

As described below with respect to FIGS. 5-16, the biostimulator 100 can include anti-dislodgement features to enhance fixation and resist dislodgement of the biostimulator from the target tissue. The anti-dislodgment features can be incorporated on or integrated with one or more of the sheath 412, the fixation elements (e.g., fixation element 106 and/or tip electrode 401), or the fixation element mount 311. In any case, the anti-dislodgement feature can resist relative motion between the biostimulator 100 and the target tissue after implantation.

Referring to FIG. 5, a side view of a distal portion of a biostimulator having a ramped protrusion is shown in accordance with an embodiment. The anti-dislodgement design at the distal end of the biostimulator 100 can include protruding features that allow the biostimulator to be easily torque into the target tissue, and resist torquing out the biostimulator. In an embodiment, the sheath 412 includes one or more protrusions 502 extending radially outward from an outer sheath surface 504. Each protrusion 502 can be a bump, boss, or other raised surface feature, which extends radially above the outer sheath surface 504 relative to the longitudinal axis 304. The protrusions 502 can have asymmetric geometric features to provide the preferential rotational direction of the pacing element 108 in the target tissue.

Referring to FIG. 6, a perspective view of a distal portion of a biostimulator having a ramped protrusion is shown in accordance with an embodiment. The asymmetric geometric features can include a raised surface having edges that providing different degrees of resistance to moving against tissue. In an embodiment, the one or more protrusions 502 include a ramped protrusion 602 having a tapered face 604 at one edge and a steeper face at another edge. The ramped protrusion 602 can have an outer protrusion surface facing radially outward from the longitudinal direction 606. For example, the outer protrusion surface may be raised above the outer sheath surface 504 that is laterally around the protrusion 502. Each edge of the outer protrusion surface can have a corresponding sidewall extending radially inward to the outer sheath surface 504. For example, the faces can include the tapered face 604 extending from a first lateral edge, e.g., a rightmost edge of the outer protrusion surface, and a steep face extending from a second lateral edge, e.g., a leftmost edge of the outer protrusion surface.

The tapered face 604 and the steep face may form different angles relative to the outer protrusion surface. The tapered face 604 may have a gradual taper, e.g., at an angle of less than 60 degrees, relative to the outer protrusion surface. By contrast, the steep face can have a steep angle, e.g., an angle of more than 60 degrees relative to the outer protrusion surface. For example, the steep angle may be perpendicular or undercut. As a result, when the pacing element 108 is rotated throughout tissue in the clockwise direction, the tapered angle can glide through the tissue and the pacing tip 402 can advance into the septal wall 110. By contrast, when the pacing element 108 is rotated in the counterclockwise direction, the steep angle can bite into the tissue and resist motion. Accordingly, the ramped protrusion 602 can facilitate implantation into the target tissue and resist dislodgement from the target tissue.

The faces of the protrusion 502 may also be angled to promote forward movement through the target tissue and resist backward movement. For example, a leading face at a front edge of the protrusion 502 can have a gradual taper, similar to the tapered face 604, and a trailing face at a rear edge of the protrusion 502 can be sharply angled or orthogonal similar to the steep face. The pacing element 108 can therefore easily advance through tissue and can resist proximal dislodgement after implantation.

The one or more protrusions 502 can include several protrusions 502, 602 arranged circumferentially about the outer sheath surface 504, as shown in FIG. 6. For example, the pacing element 108 can include several, e.g., four, discrete protrusions 502 circumferentially separated by a uniform angle, e.g., 90 degrees. In an embodiment, however, the several protrusions 502, 602 are staggered in a longitudinal direction 606 along the outer sheath surface 504. For example, one or more protrusions 502, 602 can be sequentially arranged such that an axis extending in the longitudinal direction 606 along the outer sheath surface 504 can pass through several protrusions 502. Alternatively, the protrusions 502, 602 can be staggered along a helical path 608. For example, a first protrusion can be at a first longitudinal location and a first radial location on the outer surface of the sheath 412, and a second protrusion can be at a second longitudinal location and a second radial location offset both longitudinally and circumferentially from the first protrusion. More particularly, the protrusions 502 can be repeated and staggered down a length of the pacing element 108. Accordingly, torque-out resistance may be enhanced by engaging separate tissue with the staggered protrusions 502.

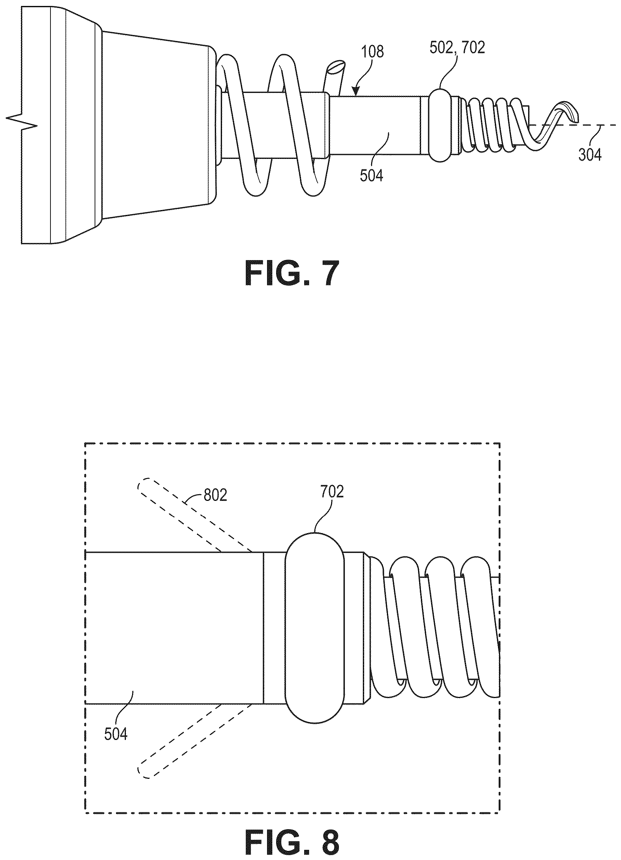

Referring to FIG. 7, a side view of a distal portion of a biostimulator having a bulbous ring is shown in accordance with an embodiment. The protrusions 502 can resist one or more of rotational or translational movement. For example, the rear edge of the protrusion 502 can be angled to engage tissue in a rearward direction and resist backout of the pacing element 108. In an embodiment, the one or more protrusions 502 include a bulbous ring 702 to resist translational (e.g., backward) motion. The bulbous ring 702 can extend circumferentially about the longitudinal axis 304. The bulbous ring 702 can have a raised surface, radially above the outer sheath surface 504, to press outward against tissue and resist movement.

Referring to FIG. 8, a side view of a distal portion of a biostimulator having a bulbous ring is shown in accordance with an embodiment. The bulbous ring 702 may have an arc-shaped outer surface. In cross-section, the bulbous ring 702 may have a semi-circular or elliptical section shape. The bulbous portion may be advantageous by locking in the bulbous portion within contracting muscle when a myocardium contracts around the target tissue. The squeezing between the contracting tissue and the bulbous ring 702 can resist translation.

It will be appreciated that protrusions 502 on the outer sheath surface 504 may not be limited to ramped shapes or bulbs. Alternative protruding features includes features such as fins or bumps to resist movement of the pacing element 108 relative to the target tissue after implantation. In an embodiment, the one or more protrusions 502 include several tines 802 extending radially outward from the outer sheath surface 504. The tines 802 may, for example, extend along radial axes, or along axes directly laterally outward and, optionally, proximally relative to the outer sheath surface 504. The protrusions 502 can be placed down a length of the pacing element 108, as single features or repeated features.

The one or more protrusions 502 can be formed separately or integrated into the sheath 412. For example, the protrusions 502 can be molded into the sheath 412, or formed by reflowing the sheath material, such as in a tipping process. Alternatively, the protrusions 502 may be attached to the outer sheath surface 504, e.g., as independent protrusions that are bonded on the surface, or as portions of a collar that is mounted on the outer sheath surface 504. For example, the tines 802 may be filaments that are embedded in the outer sheath surface 504 or silicone prongs that radiate from a collar ring placed around the outer sheath surface 504.

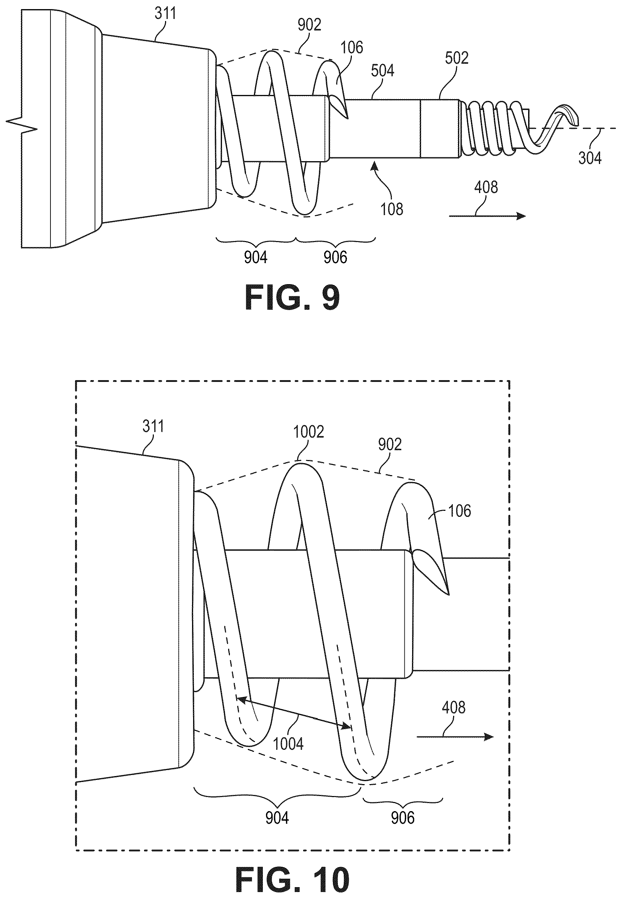

Referring to FIG. 9, a side view of a distal portion of a biostimulator having a bulging fixation element is shown in accordance with an embodiment. The fixation element 106, which is mounted on the fixation element mount 311, can have an outer dimension 902 that is shaped to resist relative motion between the fixation element 106 and the target tissue. In an embodiment, the outer dimension 902 increases and decreases over a length of the fixation element 106, forming several diverging and converging sections. For example, the fixation element 106 can include a proximal section 904 having a diverging outer dimension 902 in a distal direction 408, and a distal section 906 having a converging outer dimension 902. The variation in outer dimension 902 in the distal direction 408, e.g., the initially increasing and subsequently decreasing dimension, can provide an interior space to capture tissue and resist movement. More particularly, tissue can be sandwiched internal to the fixation element 106, both within the larger turns of the helical fixation element 106 and longitudinally between the smaller turns, to increase a grip between the fixation element 106 and tissue, and therefore resist backout of the biostimulator 100.

Referring to FIG. 10, a side view of a distal portion of a biostimulator having a bulging fixation element is shown in accordance with an embodiment. The outer dimension 902 extends over an outer profile denoted by dotted lines. In an embodiment, the outer dimension 902 increases in the distal direction 408 over the proximal section 904 of the fixation element 106. At a proximal location of the proximal section 904, e.g., at a distal end of the fixation element mount 311, the outer dimension 902 can be smaller than at a distal location of the proximal section 904. More particularly, the fixation element 106 can diverge to an apex 1002 at which the outer dimension 902 is larger than at the fixation element mount 311.

The proximal section 904 can meet the distal section 906 at the apex 1002. More particularly, the distal section 906 can be a section of the fixation element 106 that is distal to the apex 1002. The outer dimension 902 can decrease in the distal direction 408 over the distal section 906 of the fixation element 106. For example, the outer dimension 902 can be at a maximum at the apex 1002, where the proximal and distal sections 906 meet, and can converge distally to a distal tip of the fixation element 106.

The changes to the geometric shape of the fixation element 106, e.g., the diverging-converging profile of the helical fixation element 106 can provide an irregular path for the wire of the helix to travel during torque-in and torque-out. The irregular path may increase fixation and resistance to dislodgement. The irregular path may be provided, for example, by the helical wire diverging to capture more tissue between the helix and the outer sheath surface 504, and then converging to hold the captured tissue longitudinally between the distal tip and the fixation element mount 311. An overall effect of the varied helix shape can be anti-dislodgement of the biostimulator 100.

A spacing between adjacent regions of the fixation element 106 may also provide anti-dislodgement advantage. In an embodiment, the fixation element 106 includes a helical pitch 1004 between adjacent turns of a helical wire. The helical pitch 1004 may be varied in the distal direction 408 to provide some torque-out resistance. For example, the distal coils may contain a wider pitch with the pitch shortening proximally to compress or pull the tissue during fixation. More particularly, the helical pitch 1004 can increase in the distal direction 408. The pitch variation and/or tapering helical fixation element 106 provide a mechanical advantage to tissue gripping. For example, it has been shown that the tapering fixation element 106 can increase, by over 40%, a pull force required to dislodge the biostimulator 100 from tissue, when compared to a helical fixation element having a constant outer dimension over the fixation element length.

In addition to enhancing mechanical grip of the target tissue, the pitch that is tighter at a base of the fixation element 106, near the fixation element mount 311, may provide visual advantage. For example, the tighter pitch, or variation in pitch, may be visible under fluoroscopy. Accordingly, a physician may view the fixation element 106 to determine when the helix pitch expands during delivery. The expansion can indicate that the fixation element 106 has properly engaged the target tissue.

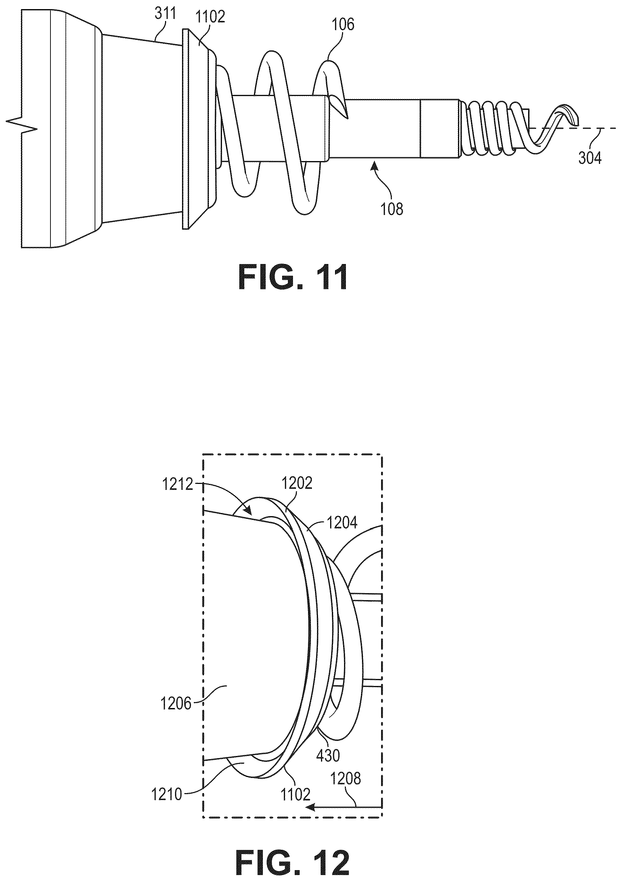

Referring to FIG. 11, a side view of a distal portion of a biostimulator having a compliant ring is shown in accordance with an embodiment. The anti-dislodgement features may be located on the fixation element mount 311. For example, the fixation element mount 311 can include a compliant ring 1102, shaped to engage surrounding tissue, which extends circumferentially about the longitudinal axis 304. The compliant ring 1102 can increase an interaction with surrounding cardiac tissue. The compliant ring 1102 can be a pliable, directional ring that allows ease of movement in one direction, e.g., distally, and resists motion in an opposite direction, e.g., proximally. Accordingly, the compliant ring 1102 can resist dislodgement of the biostimulator 100 from the target tissue.

Referring to FIG. 12, a perspective view of a distal portion of a biostimulator having a compliant ring is shown in accordance with an embodiment. The compliant ring 1102 can include an annular flange 1202 extending around the fixation element mount 311. The annular flange 1202 can have an outer flange surface 1204 facing radially outward from the fixation element mount 311. For example, the fixation element mount 311 can have an outer mount surface 1206, and the outer mount surface 1206 and outer flange surface 1204 can both face in an outward direction, away from the longitudinal axis 304. The annular flange 1202 can extend circumferentially about the outer mount surface 1206. For example, the annular flange 1202 can include a ring-like band of material mounted on the fixation element mount 311 at or near the distal mount end 430.

In an embodiment, the outer flange surface 1204 of the annular flange 1202 tapers in a proximal direction 1208, e.g., opposite to the distal direction 408. The flange 308 can taper outward, and may be formed from a band of material providing a flap that defines a space between the flange 308 and the outer mount surface 1206. For example, the annular flange 1202 can have an underside 1210, opposite to the outer flange surface 1204, that faces inward toward the outer mount surface 1206 across a gap. The gap can include a void 1212, located between the underside 1210 of the annular flange 1202 and the outer mount surface 1206 of the fixation element mount 311.

The compliant ring 1102 can extend entirely circumferentially about the outer mount surface 1206, or could include one or more arc sections that are circumferentially separated from each other, e.g., forming compliant arc sections. In any case, the pliable flaps of material provide anti-dislodgement for several reasons. First, the outer flange surface 1204 can press outward against tissue to increase a back pressure placed on the fixation element 106. The increased back pressure can increase an anchoring force in the target tissue. The front face of the compliant ring 1102 can engage the tissue, and the flap of material can provide spring force as the band flexes into the void 1212, creating a reaction force to the tissue that increases the back pressure. Additionally, the underside 1210 of the annular flange 1202 may act like a barb, and grip tissue or cardiac structures such as trabeculae, chordae tendineae, etc. within the void 1212. Accordingly, a likelihood of dislodgement of the biostimulator 100 can be reduced by enhancing anchoring of the fixation element 106 and by gripping the target tissue by the compliant ring 1102.

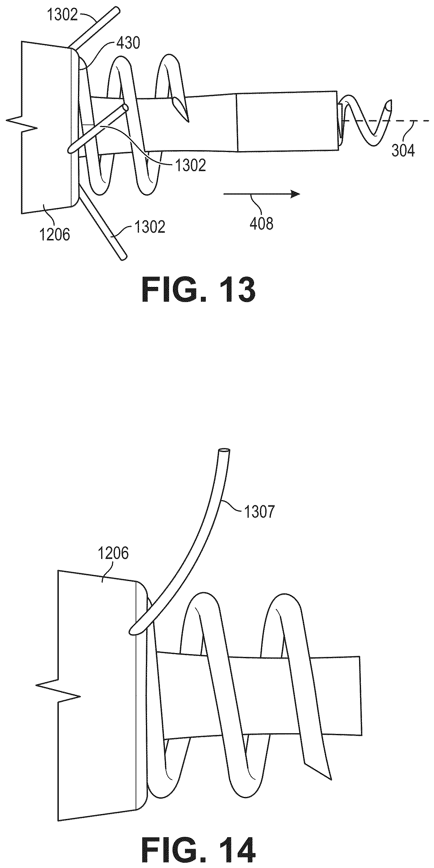

Referring to FIG. 13, a side view of a distal portion of a biostimulator having a flexible barb is shown in accordance with an embodiment. The anti-dislodgement features can, as described above, include tines or barbs that extend outward from an outer surface of the biostimulator 100. In an embodiment, the features can extend from the fixation element mount 311. For example, the fixation element mount 311 can include several flexible barbs 1307 extending from the outer mount surface 1206. The barbs can extend in the distal direction 408. Accordingly, the barbs can extend distal to the distal mount end 430 to engage tissue that the fixation element mount 311 engages during implantation.

The fixation barbs can include filaments, whiskers, or another flexible feature that protrudes from the fixation element mount 311 and angles up toward the distal end of the device. For example, the barbs can include suture doped with radiopaque material, thin metal cables, or other flexible and radiopaque structures. The angulated barbs can make contact with the tissue prior to the most distal extent of the fixation mount engaging the tissue. Accordingly, the flexible barbs 1307 can be bent backward to allow the distal mount end 430 of the fixation element mount 311 to advance into contact with the septal tissue.

Referring to FIG. 14, a side view of a distal portion of a biostimulator having a flexible barb is shown in accordance with an embodiment. There may be any number of barbs, e.g., three or more, incorporated in the header assembly of the biostimulator 100 to increase a force required for anti-rotation. The flexible barbs 1307 may assist with fixation, and may also assist with visualization of depth of the biostimulator 100 at implant. For example, the flexible barbs 1307 can include a radiopaque material. The barbs may be doped with the radiopaque material, e.g., barium sulphate, titanium dioxide, etc., which is visible under fluoroscopy. The barbs can be flexible and, accordingly, when the barbs contact tissue the barbs can deflect.

Deflection of the barbs can be viewed as the barbs bending or curving backward, as shown in FIG. 14. The deflected shape can be compared to the normally straight shape of the barbs shown in FIG. 13. More particularly, a change in shape of the barbs can be viewed and used to determine that the barbs have engaged tissue, e.g., the septal wall 110. Accordingly, when the tissue is engaged, the barbs can fold back to give feedback regarding a location of a header assembly of the biostimulator 100.

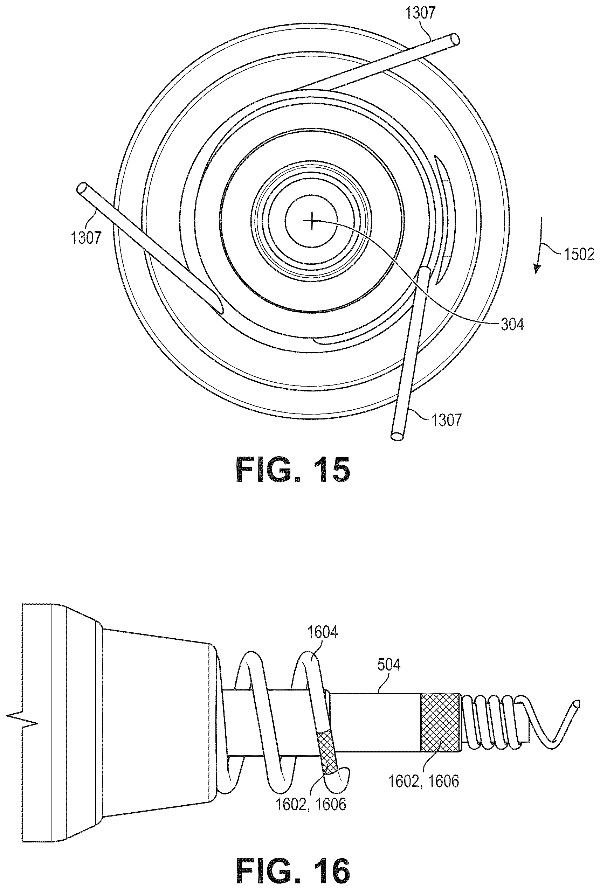

Referring to FIG. 15, an end view of a distal portion of a biostimulator having a flexible barb is shown in accordance with an embodiment. The flexible barbs 1307 can extend radially and be angled between tangent to a circumference of the fixation element mount 311 and normal to the circumference. More particularly, the flexible barbs 1307 can extend in a rotational direction 1502. For example, the barbs can extend in a clockwise direction about the longitudinal axis 304. The rotational and longitudinal angle of the barbs can allow the barbs to freely slide over the target tissue when the biostimulator 100 is rotated in a first direction, e.g., clockwise, and to engage the tissue when the biostimulator 100 is rotated in an opposite direction, thereby resisting backout of the biostimulator 100 and requiring more force to cause the biostimulator 100 to be removed from the tissue.

Referring to FIG. 16, a side view of a distal portion of a biostimulator having a rough surface is shown in accordance with an embodiment. The biostimulator 100 can include one or more modified or rough surfaces 1602 to enhance fixation and resist dislodgement from the target tissue. In an embodiment one or more of the sheath 412 or the fixation element 106 have a rough surface 1602. The rough surface 1602 can be provided by a coating, a texture, a fabric embedded or wrapped over a surface of the biostimulator 100, etc. Rough surface 1602 may combine roughening techniques. For example, the surface may be laser roughened and a fabric may be embedded or wrapped over the surface coincident or adjacent to the laser roughening. In any case, the rough surface 1602 can provide friction to the tissue to improve fixation and resist dislodgement of the biostimulator 100.

In an embodiment, the sheath 412 has the rough surface 1602. For example, the outer sheath surface 504 can be roughened or otherwise surface modified to create a surface roughness that is greater than a surrounding sheath surface region. More particularly, the outer sheath surface 504 can be roughened to resist relative movement between the pacing element 108 and the target tissue.

In an embodiment, the fixation element 106 has the rough surface 1602. For example, an outer element surface 1604 of the fixation element 106 can be roughened or otherwise surface modified to create a surface roughness that is greater than a surrounding fixation element 106 region. More particularly, the outer sheath surface 504 can be roughened to resist relative movement between the pacing element 108 and the target tissue.

The surface roughness may be caused by laser roughening of the surface. For example, the outer sheath surface 504 or the outer element surface 1604 can be laser roughened to create directional protrusion 502 features, e.g., barbs, at a macro scale. The laser roughened surface can increase frictional interactions in one direction or several directions. More particularly, the roughened surface of the pacing element 108 or the fixation element 106 can resist rotation relative to tissue and thereby provide an anti-dislodgement feature. The surface roughness may be provided by embedding rough materials in the biostimulator components. For example, the rough surface 1602 can include a woven fabric 1606 embedded in the outer sheath surface 504 of the sheath 412. The woven fabric 1606 can include a biocompatible open weave fabric, e.g., Kevlar, which is embedded in the most outer surface of the pacing element 108 to increase roughness and increase friction. In an embodiment, the woven fabric 1606 includes a tubular element or a sheet of fabric that is wrapped or otherwise mounted on the outer sheath surface 504 and adhered, e.g., by a chemical or thermal adhesion process. The fabric can increase friction and resist dislodgement of the biostimulator 100 from the target tissue.

The woven fabric 1606 may alternatively be loaded onto the fixation element 106. For example, the rough surface 1602 can include the woven fabric 1606, e.g., a woven tubular element, on the outer element surface 1604 of the fixation element 106. The woven fabric 1606 can be adhered to the helix, for example. Accordingly, when the fixation element 106 is screwed into the target tissue, the rough surface 1602 can resist backing out and dislodgement of the biostimulator 100.

Roughening of the biostimulator surfaces can be performed by alternative processes. For example, particles may be embedded in the component surfaces. In an embodiment, particles are loaded in an outer surface of the polymer surfaces of the biostimulator 100, such as the outer sheath surface 504, to provide the frictional interaction with cardiac tissue described above. Accordingly, anti-dislodgement features can be directly incorporated into the biostimulator 100 surfaces to enhance fixation of the biostimulator 100 the target tissue.

In the foregoing specification, the invention has been described with reference to specific exemplary embodiments thereof. It will be evident that various modifications may be made thereto without departing from the scope of the invention as set forth in the following claims. The specification and drawings are, accordingly, to be regarded in an illustrative sense rather than a restrictive sense.

## Claims

1. A biostimulator (100), comprising:
a housing (302) having a longitudinal axis (304) and containing an electronics compartment (306);
a fixation element mount (311) mounted on the housing (302); and
a fixation element (106) mounted on the fixation element mount (311), wherein the fixation element (106) extends helically about the longitudinal axis (304) and has an outer dimension (902), wherein the outer dimension (902) increases in a distal direction (408) over a proximal section (904) of the fixation element (106), and wherein the outer dimension (902) decreases in the distal direction (408) over a distal section (906) of the fixation element (106).

2. The biostimulator of claim 1, wherein the proximal section (904) and the distal section (902) meet at an apex (1002).

3. The biostimulator of claim 2, wherein the outer dimension (902) of the fixation element (106) is at a maximum at the apex (1002).

4. The biostimulator of claim 2 or 3, wherein the fixation element (106) diverges to the apex (1002) at which the outer dimension (902) is larger than at the fixation element mount (311).

5. The biostimulator of any one of claims 1 to 4, wherein the fixation element (106) has a helical pitch (1004) that varies in the distal direction (408).

6. The biostimulator of claim 5, wherein the proximal section (904) of the fixation element (106) is more visible under fluoroscopy than the distal section (906) of the fixation element (106).

7. The biostimulator of claim 5 or 6, wherein the helical pitch (1004) increases in the distal direction (408).

8. The biostimulator of any one of claims 1 to 7, wherein variation in the outer dimension (902) over a diverging-converging profile of the fixation element (106) defines an interior space configured to capture tissue.

9. The biostimulator of any one of claims 1 to 8, wherein the fixation element mount (311) includes a plurality of flexible barbs (1307) extending in the distal direction (408).

10. The biostimulator of claim 9, wherein the plurality of flexible barbs (1307) extend in a rotational direction (1502).

11. The biostimulator of claim 9 or 10, wherein the plurality of flexible barbs (1307) include a radiopaque material.

12. The biostimulator of any one of claims 1 to 11, further comprising:
a pacing element (108) coupled to the housing (302), wherein the pacing element (108) includes a conductor (410) extending along the longitudinal axis (304) to a pacing tip (402), and a sheath (412) surrounding the conductor (410), wherein the sheath (412) has a rough surface (1602).

13. The biostimulator of claim 12, wherein the rough surface (1602) is laser roughened.

14. The biostimulator of claim 12 or 13, wherein the rough surface (1602) includes a woven fabric (1606) embedded in an outer sheath surface (504) of the sheath (412).

15. A biostimulator system (200), comprising:
a biostimulator transport system (202); and
the biostimulator (100) of any one of claims 1-14 mounted on the biostimulator transport system (202).
